# EUROPEAN PATENT APPLICATION

(11) **EP 2 335 601 A2**
(43) Date of publication of application: **22.06.2011**
(21) Application number: 10190991.9
(22) Date of filing: 06.10.2003
(51) Int. Cl.: A61B 17/02

(54) **Endoscopic retractor**

(30) Priority: 04.10.2002 US 416370 P
(62) Divisional of application: 03808155.0
(71) Applicant: Tyco Healthcare Group LP, Norwalk, CT 06856 (US)
(72) Inventor: Stearns, Ralph, A., Bozrah, CT 06334 (US); Orban, Joseph, P., III, Norwalk, CT 06850 (US); Viola, Frank, J., Sandy Hook, CT 06482 (US)
(74) Representative: Soames, Candida Jane

(57) **Abstract**

An organ retractor, comprising a shaft defining a longitudinal axis and a bore for receiving a temperature changing medium. The shaft is fabricated from a shape memory substance. The shaft has a first configuration which is substantially linear when at a first temperature and at least one second configuration which is non-linear when at a second temperature.

## Description

### CROSS-REFERENCE TO RELATED APPLICATIONS

The present application claims the benefit of and priority to U.S. Provisional Application Serial No. 60/416,370, filed on October 4, 2002, the entire content of which is incorporated herein by reference.

### BACKGROUND

### 1. Technical Field

The present disclosure relates to endoscopic surgical retractors and organ manipulators for use during minimally invasive surgical procedures and, more particularly, to endoscopic surgical retractors which are configurable from a first position for insertion through a trocar or surgical sheath to a second position to facilitate retraction and/or manipulation of organs or tissue.

### 2. Background of Related Art

It is well established that the performance of various types of surgical procedures using minimally invasive techniques and instrumentation has provided numerous physical benefits to the patient while reducing the overall cost of such procedures. Endoscopic surgical procedures have been used for many years and the popularity of such procedures continues to increase. For example, more and more surgeons are complementing traditional open methods of gaining access to vital organs and body cavities with endoscopes and endoscopic instruments which access organs through small puncture-like incisions.

Once inserted into the initial incision, a trocar provides a narrow passageway for endoscopic instruments which are inserted into the patient through a cannula or port disposed within the trocar. As can be appreciated, access to the surgical cavity is typically limited to the internal dimensions of the trocar channel and the size of the cannula. It is believed that minimizing the size of the incision minimizes pain and provides other benefits to the patient. Smaller cannulas are usually preferred during most endoscopic procedures which, ultimately, present a design challenge to instrument manufacturers who must find ways to make surgical instruments which will fit through the small cannulas.

For example, it is known that the ability to manipulate organs and tissue within the operating cavity is important during endoscopic procedures due to the limited view within the operating cavity. Utilizing a traditional open retractor to manipulate organs and/or retract tissue is not an option since it would force a surgeon to forego the benefits of minimally invasive surgery. As a result, manufacturers have been challenged to design various types of endoscopic retractors which can fit through small cannulas and which do not limit a surgeon's ability to retract and/or manipulate organs and tissue as needed during the surgical procedure.

Several endoscopic retractors have been designed in the past but for the most part and by and large these instruments are overly complex and only allow relatively limited positioning or repositioning of organs during surgery. A need exists to develop a retractor which is simple and effective in manipulating organs to provide adequate visualization of the operating cavity for the surgeon during endoscopic surgical procedures.

### SUMMARY

The present disclosure relates to endoscopic retractors for retracting organs and the like. According to one aspect of the present disclosure, a retractor includes a shaft having at least a first section having a first mechanical interface and a second section having a second mechanical interface for engaging the first mechanical interface, the first section and the second section being selectively movable from a first, generally longitudinally-aligned configuration along an axis defined through the shaft and the first mechanical interface is disengaged from the second mechanical interface, to a second configuration wherein the second section is disposed at an angle relative to a longitudinal axis of the shaft and the first mechanical interface is engaged with the second mechanical interface. The retractor further includes at least one cable extending through the shaft and is operatively secured to the second section. The cable is remotely actuatable to move the second section from the first to the second configuration upon selective translation of the cable.

The first and second mechanical interfaces desirably cooperate to align the first section and the second section and engage the first section and second section with one another upon movement from the first configuration to the second configuration.

The first and second sections can include complementary cam-like interfaces. The cam-like interfaces rotatably and translatably engage one another upon actuation of the cable for movement from the first configuration to the second configuration. Accordingly, the first section and the second section can rotate and translate with respect to one another. The shaft can include an outer sleeve which houses the first and second sections.

At least one of the first section and the second section can include a tongue which engages a corresponding recess disposed within the other of the first section and the second section. The tongue desirably facilitates alignment and engagement of the first section and the second section relative to one another during movement from the first configuration to the at least one additional second configuration.

The retractor may include a hinge disposed between the first section and the second section. In one embodiment, the hinge is a living hinge disposed between the first section and the second section. It is envisioned that one of the first section and the second section can include a stop for controlling the angular disposition of the first section and the second section when disposed in the at least one additional second configuration.

According to another aspect of the present disclosure, the organ retractor includes a tube having a lumen extending therethrough and defining a longitudinal axis, and a distal section, an intermediate section and a proximal section disposed within the lumen of the tube. The retractor has a first configuration in which the distal, intermediate and proximal sections are substantially aligned with the longitudinal axis and disassociated with one another, and at least one second configuration in which the intermediate section and the distal section are engaged with one another so that the distal section is disposed at an angle with respect to the longitudinal axis. The tube is desirably formed from a flexible material.

The organ retractor can further include a first cable extending through the proximal section and the intermediate section, and operatively secured to the distal section. Accordingly, translation of the first cable in a proximal direction causes the distal section to operatively engage the intermediate section at an angle relative to the longitudinal axis. The organ retractor can further include a second cable extending through the proximal section and operatively secured to the intermediate section. Accordingly, translation of at least one of the first and second cables in a proximal direction causes the intermediate section to operatively engage the proximal section.

The distal section can include at least one first mechanical interface, formed at a proximal end thereof and the intermediate section can include at least one second mechanical interface formed on a side surface thereof The second mechanical interface is complementary with the first mechanical interface. Accordingly, when the distal and intermediate sections engage one another, the second mechanical interface and the first mechanical interface maintain the distal section at an angle with respect to the longitudinal axis.

The proximal section includes at least one third mechanical interface formed at a distal end thereof and the intermediate section includes at least one fourth mechanical interface formed at a proximal end thereof. The fourth mechanical interface is preferably complementary to the third mechanical interface. Accordingly, when the proximal and intermediate sections engage one another, the third mechanical interface and the fourth mechanical interface maintain the proximal and intermediate sections substantially aligned with the longitudinal axis.

The proximal section preferably includes at least one longitudinally oriented passage extending therethrough, wherein the first and the second cables extend through the at least one longitudinal passage. The intermediate section preferably includes a substantially angular passage extending therethrough. A first portion of the angular passage opens on the proximal surface of the intermediate section and a second portion of the angular passage opens on the side surface of the intermediate section. The second cable extends through the angular passage.

It is envisioned that the second mechanical interface of the intermediate section can be in the form of a socket and the first mechanical interface of the distal section can be in the form of a tongue-like member which extends therefrom and is complementary to the socket formed in the proximal section.

The organ retractor can include a cable in the form of a ribbon extending through the proximal section and the intermediate section and which is affixed to the distal section. It is envisioned that the tube can be fabricated from an elastic material.

In one embodiment, the second mechanical interface of the intermediate section includes a helical camming surface and the first mechanical interface of the distal section includes a helical camming surface which is complementary to the helical camming surface of the proximal section. In another embodiment, the third mechanical interface of the proximal section and the fourth mechanical interface of the intermediate section each include a helical camming surface which intersect one another.

According to another aspect of the present disclosure, the organ retractor includes an elongated shaft defining a longitudinal axis, the shaft having a first section and a second section pivotably connected to one another, and a first cable extending through the first section and operatively connected to the second section. The first cable is used to manipulate the retractor from a first configuration to at least one second configuration. In the first configuration the first and second sections are substantially aligned with the longitudinal axis while in the at least one second configuration the second section is at an angle with respect to the longitudinal axis. The second section may be pivotably connected to the first section by a mechanical hinge and/or a living hinge.

It is envisioned that the first section has a distal surface and the second section has a proximal surface, the distal surface comprising an angled surface that faces the proximal surface of the second section. The organ retractor can further include a film extending between the first and second sections. The organ retractor can further include at least one stop member provided on at least one of the distal surface and the proximal surface.

In another embodiment, the organ retractor further includes a third section pivotably connected to the second section, and a second cable extending through the first section and the second section and operatively connected to the third section for manipulating the retractor from the first configuration to the at least one second configuration. The organ retractor can further include a first mechanical interface provided on the first section, a second mechanical interface provided on the second section for engaging the first mechanical interface, a third mechanical interface provided on the second section, and a fourth mechanical interface on the third section for engaging the third mechanical interface.

According to a further aspect of the present disclosure, the organ retractor includes a shaft defining a longitudinal axis, and a plurality of finger elements operatively engagable with a distal end of the shaft. The retractor preferably has a first configuration in which the plurality of finger elements are substantially aligned with the longitudinal axis and at least one second configuration in which the plurality of finger elements are disposed at an angle with respect to the longitudinal axis.

It is envisioned that each of the plurality of finger elements is disassociated from the shaft, and wherein the retractor includes a plurality of cables extending through the shaft, each cable having a bundle of cords extending therefrom and into a corresponding finger element. Each bundle of cords is operatively connected to the corresponding finger element such that retraction of the plurality of cables manipulates the retractor from the first configuration to the at least one second configuration. It is envisioned that the bundle of cords can extend between the plurality of finger elements.

In one embodiment, a distal end of the shaft can include a plurality of sockets configured and dimensioned to selectively receive a flange formed at a proximal end of a corresponding finger element. The individual cords of the bundle of cords can exit a respective finger element through ports formed therein.

In another embodiment, the organ retractor can further include a pair of plates pivotably connected to a distal end of the shaft. The plurality of finger elements can preferably be affixed to the pair of plates. The pair of plates can have a first orientation in which the retractor is in the first configuration and a second orientation in which the retractor is in the at least one second configuration. The organ retractor can further include at least one wire extending between adjacent finger elements.

According to yet another aspect of the present disclosure, the organ retractor includes a shaft defining a longitudinal axis and a bore for receiving a temperature changing medium. The shaft is desirably fabricated from a shape memory substance. The shaft has a first configuration which is substantially linear when at a first temperature and at least one second configuration which is non-linear when at a second temperature. In one embodiment, the temperature changing medium includes a quantity of liquid received in the bore.

It is envisioned that the shaft can be fabricated from a shape memory alloy and/or a shape memory plastic. Preferably, the shaft is fabricated from nitinol. The shaft will desirably undergo a change of configuration from about -270°C to about +100°C. It is envisioned that the liquid can transmit a change of temperature to the shaft to effectuate the change in configuration.

According to another aspect of the present disclosure, a retractor is provided including a plurality of sections defining a shaft, each of the sections having a mechanical interface for engaging an adjacent section, each section having a first position in longitudinal aliment with an adjacent section and a second position offset from the first position so that the sections form a substantially closed shape for engaging tissue.

In one embodiment, at least one of the sections includes a tongue for engaging a slot in an adjacent section. In another embodiment, a first cable can be attached to at least a first section of the plurality of sections and disposed in a passage in at least a second section of the plurality of sections, and arranged for moving the first section with respect to a second section when the first cable is pulled in a proximal direction, the first cable being offset from a longitudinal axis of the shaft in a first direction. The retractor can further include a second cable offset from the longitudinal axis in a second direction, for returning the retractor to the first position. In yet another embodiment, the retractor includes a hinge, desirably a living hinge, disposed between a first section of the plurality of sections and a second section of the plurality of sections.

### BRIEF DESCRIPTION OF THE DRAWINGS

Other objects and features of the present disclosure will become apparent from the following detailed description considered in connection with the accompanied drawings. It should be understood, however, that the drawings are designed for the purpose of illustration only and not as a definition of the limits of the disclosure.

Illustrative embodiments of the subject surgical instrument are described herein with reference to the drawings wherein:
FIG. 1A is a side sectional view of an endoscopic retractor constructed in accordance with one embodiment of the present disclosure shown configured for insertion through a trocar assembly;
FIG. 1B is a side sectional view of an retractor in accordance with the embodiment of FIG. 1A showing a first stage of deployment wherein a third section of the retractor is rotated into position for retracting tissue;
FIG. 1C is a side sectional view of an retractor in accordance with the embodiment of FIGS. A and 1B showing a second stage of deployment wherein a second section of the retractor is translated to engage and lock against a first section of the retractor to position the retractor for retracting tissue;
Fig. 1D is a perspective view of a retractor in accordance with another embodiment wherein a chamfered tongue-like fitting is utilized to facilitate engagement of the second section and the first section to one another;
FIG. 1E is a cross-sectional view, taken along line 1E - 1E in FIG. 1D, showing a pair of cables which are used to remotely translate the second section relative to the first section;
FIG. 1F is an cross-sectional view, taken along line 1F - 1F in FIG. 1D, showing a ribbon-like cable which is used to remotely translate the second section relative to the first section;
FIG. 1G is a perspective view of a mechanical interface in a retractor in accordance with a further embodiment, showing a first section and a second section;
FIG. 1H is a perspective view showing the mechanical interface in accordance with the embodiment of FIG. 1G, showing the second section in a different position;
FIG. 1I is a perspective view of a first section in accordance with an alternate embodiment;
FIG. 1J is a perspective view illustrating a first section and second section in accordance with the embodiment of FIG. 1I;
FIG. 2A is a side elevational view of an endoscopic retractor in accordance with another embodiment of the present disclosure;
FIG. 2B is a side elevational view of an endoscopic retractor in accordance with the embodiment of FIG. 2A showing the retractor positioned for retracting tissue;
FIG. 2C is a side elevational view of an endoscopic retractor in accordance with a further embodiment having three sections;
FIG. 2D is a front elevational view of an endoscopic retractor in accordance with a further embodiment showing a locking mechanism;
FIG. 3A is a side elevational view of an endoscopic retractor in accordance with yet another embodiment of the present disclosure;
FIG. 3B is a side elevational view of the endoscopic retractor of FIG. 3A showing the retractor positioned for retracting tissue;
FIG. 4A is a schematic side elevational view of an endoscopic organ retractor in accordance with another embodiment of the present disclosure, shown in a first or extended condition;
FIG. 4B is a schematic side elevational view of the endoscopic organ retractor in accordance with the embodiment of FIG. 4A, shown in a second or partially retracted condition;
FIG. 4C is a schematic side elevational view of the endoscopic organ retractor in accordance with the embodiment of FIGS. 4A and 4B, shown in a third or fully retracted condition;
FIG. 4D is a perspective detail view of the indicated area shown in FIG. 4A of the endoscopic organ retractor in accordance with the embodiment of FIGS. 4A - 4C;
FIG. 5A is a perspective view of a first section of an endoscopic organ retractor in accordance with still another embodiment of the present disclosure;
FIG. 5B is a perspective view of a second section of the endoscopic organ retractor in accordance with the embodiment of FIG. 5A;
FIG. 5C is a perspective view of the endoscopic retractor in accordance with the embodiment of FIGS. 5A and 5B;
FIG. 6A is a front elevational view of an endoscopic retractor in accordance with a further embodiment of the present disclosure;
FIG. 6B is a side elevational view of the endoscopic retractor in accordance with the embodiment of FIG. 6A;
FIG. 6C is a front elevational view of the endoscopic retractor in accordance with another embodiment;
FIG. 7A is a left side elevational view of an endoscopic retractor in accordance with yet another embodiment of the present disclosure;
FIG. 7B is a front elevational view of the endoscopic retractor in accordance with the embodiment of FIG. 7A;
FIG. 7C is a right side elevational view of the endoscopic retractor in accordance with the embodiment of FIGS. 7A and 7B;
FIG. 8A is a front elevational view of an endoscopic retractor in accordance with still another embodiment of the present disclosure;
FIG. 8B is a side elevational view of the endoscopic retractor in accordance with the embodiment of FIG. 8A;
FIG. 9A is a front perspective view of an endoscopic retractor in accordance with still another embodiment of the present disclosure, shown in a first or disassembled configuration, wherein a series of finger elements cooperate to retract tissue;
FIG. 9B is a front perspective view of the endoscopic retractor in accordance with the embodiment of FIG. 9A shown in a second or assembled configuration;
FIGS. 10A is a front elevational view of an endoscopic retractor in accordance with still another embodiment of the present disclosure and having a scoop-like configuration for retracting tissue, shown in a first or disassembled configuration;
FIG. 10B is a front perspective view of the endoscopic retractor in accordance with the embodiment of FIG. 10A shown in a second or assembled configuration; and
FIGS. 11A and 11B are schematic illustrations of an endoscopic retractor in accordance with another embodiment of the present disclosure, wherein FIG. 11A shows the organ retractor in a first or insertion/withdrawal configuration, and FIG. 11B shows the organ retractor in a second or retracted configuration.

### DETAILED DESCRIPTION OF PREFERRED EMBODIMENTS

In the drawings and in the description which follows, the term "proximal", as used in the technical field, will refer to the end of the surgical device or instrument of the present disclosure which is closest to the operator, while the term "distal" will refer to the end of the device or instrument which is furthest from the operator.

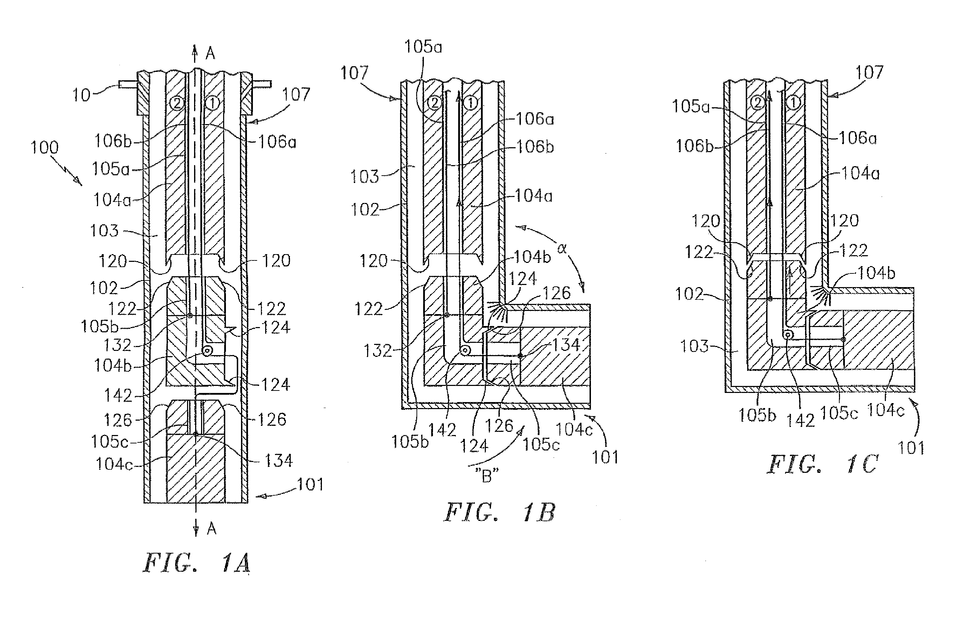

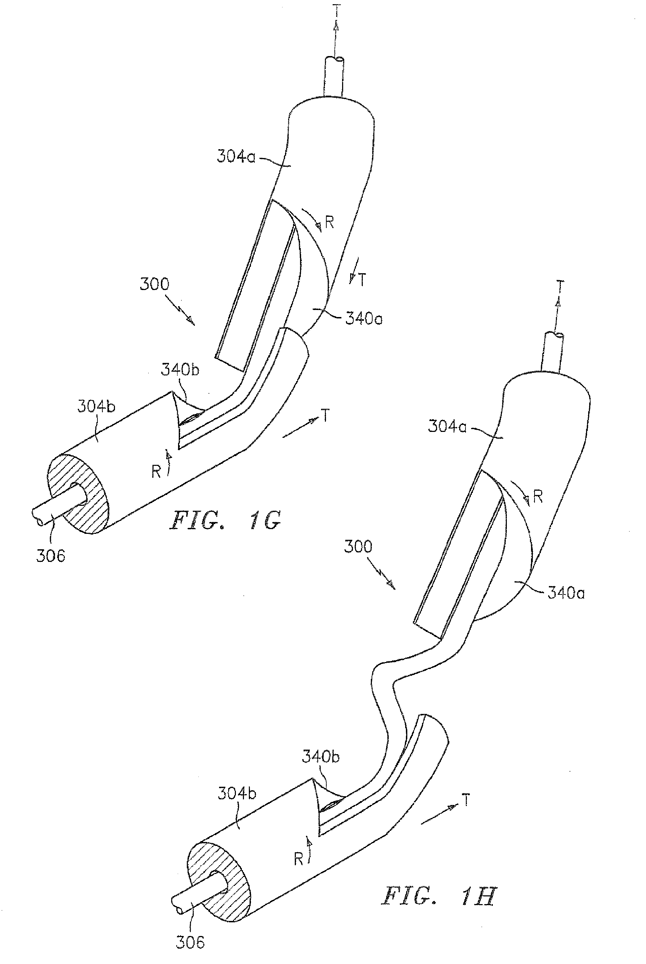

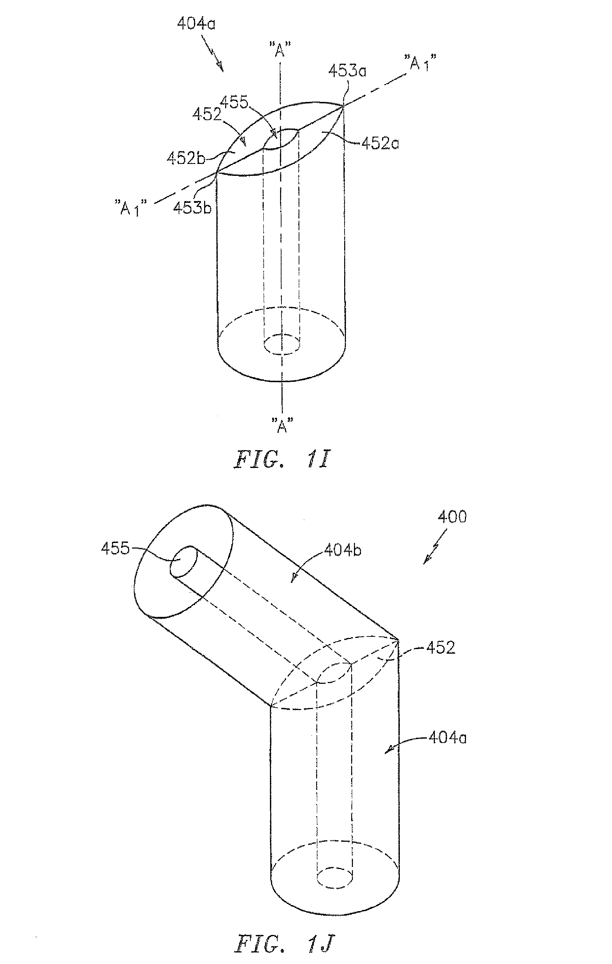

Referring now in specific detail to the drawings, in which like reference numerals identify similar or identical elements, FIGS. 1A - " 1J illustrate an endoscopic organ retractor, in accordance with an embodiment of the present disclosure, generally designated as 100.

As seen in particular in FIGS. 1A - 1C, organ retractor 100 includes an elongated tube 102, preferably flexible, having a lumen 103 defining a longitudinal axis "A" extending therethrough. Lumen 103 of tube 102 is desirably configured and dimensioned to house three interacting sections, namely, a third section 104a, a second section 104b and a first section 104c. The three sections 104a, 104b and 104c are desirably configured to be movable from a first configuration wherein sections 104a, 104b and 104c are generally aligned along longitudinal axis "A" to at least one additional second configuration wherein sections 104a, 104b and 104c engage one another and configure and/or shape retractor 100 in a manner to retract tissue. The first section 104c is disposed at a distal end 101 of retractor 100 and the third section 104a is disposed at a proximal end 107 of retractor 100.

First section 104c includes a first mechanical interface 126, formed at a proximal end 125 thereof, which is configured and dimensioned to be engagable with a corresponding and/or complementary second mechanical interface 124 desirably formed in a side surface 122 of second section 104b. Similarly, third section 104a includes a third mechanical interface 120, formed at a distal end 128 thereof, which is configured and dimensioned to be engagable with a corresponding and/or complementary fourth mechanical interface 122 desirably formed in a proximal end 130 of second section 104b.

Retractor 100 includes a first cable 106a extending through a longitudinally oriented passage 105a formed in third section 104a, into second section 104b through a proximal longitudinally oriented portion of an L-shaped passage 105b and out second section 104b through a radially oriented portion of L-shaped passage 105b, and affixed to first section 104c at an attachment point 134, preferably located in a bore 105c formed in and extending proximally out of first section 104c. Retractor 100 further includes a second cable 106b extending through passage 105a of third section 104a and affixed to second section 104b at an attachment point 132, preferably located within L-shaped passage 105b. Cables 106a and 106b are remotely translatable by the surgeon to effectuate manipulation of retractor 100 between the first and second configuration. A series of cable guides 142 may be used to facilitate translation of cables 106a and 106b through and/or into sections 104a, 104b and 104c.

As seen in FIGS. 1A - 1E, cables 106a, 106b are preferably offset a distance from longitudinal axis "A". Most preferably, at least cable 106a is offset from longitudinal axis "A".

In use and as best illustrated in FIGS. 1A - 1C, retractor 100 is initially configured as shown in FIG. 1A (e.g., sections 104a-104c substantially longitudinally aligned with one another along longitudinal axis "A") to facilitate insertion of retractor 100 through a trocar assembly 10. Once the retractor 100 is inserted a desired and/or a sufficient distance, through trocar assembly 10 into the operative site, the surgeon remotely actuates first cable 106a (e.g., pulls on first cable 106a in a proximal direction) thereby causing first section 104c to rotate in the direction of arrow "B", slide and/or be positioned into engagement with a side surface of second section 104b. More particularly, by retracting cable 106a, first section 104c is positioned against second section 104b such that the two pairs of opposing mechanical interfaces, namely, interface 124 of second section 104b and interface 126 of first section 104c, engage one another to position first section 104c in angular, preferably orthogonal orientation with respect to second section 104b. Cable 106a may be actuated after placing the distal end 101 adjacent an organ or tissue to be retracted so that the cable moves first section 104c and the organ or tissue simultaneously. Alternatively, the retractor may be moved into the second configuration and then engaged with an organ or tissue to be retracted. Then retractor 100 is moved to move the organ or tissue.

As can be appreciated, while the particular configurations of first and second sections 104c and 104b are shown at a substantially 90° angle relative to one another, it is envisioned and within the scope of the present disclosure that the orientation of first section 104c relative to second sections 104b can be at any angle "a" relative to longitudinal axis "A" (See FIG. 1B). Cable 106a may be subsequently locked to securely affix second and first sections 104b and 104c relative to one another for retraction purposes.

Once first section 104c has been engaged with intermediate section 104b, the surgeon remotely actuates second cable 106b (e.g., pulls on second cable 106b in a proximal direction) thereby causing second section 104b to translate and/or be positioned into engagement with proximal section 104a along longitudinal axis "A". In particular, as best seen in FIG. 1C, by retracting cable 106b, second section 104b is positioned against third section 104a such that the two pairs of opposing mechanical interfaces, namely, pair 120 of third section 104a and pair 122 of second section 104b, engage one another to align and secure second section 104b against third section 104a as best seen in FIG. 1C. Cable 106b maybe subsequently locked to securely affix second section 104b relative to third section 104a for retraction purposes. First section 104c, second section 104b and third section 104a may be disengaged by releasing cable 106a and 106b so that the sections return and/or are free to return to the initial configuration under the action of gravity. Retractor 100 can then be removed from trocar assembly 10.

As can be appreciated, first and second cables 106a, 106b may also be remotely actuated in reverse order, i.e., 106b actuated first followed by first cable 106a, or simultaneously depending upon a particular purpose. Interfaces 120, 122, 124 and 126 may include any combination of one or more detents, flanges, pins, tabs, grooves, slots, or the like which complement one another and which securely engage third, second and first sections 104a, 104b and 104c with one another for retraction purposes.

Preferably, flexible tube 102 is biased in a generally linear and/or straight orientation along longitudinal axis "A" and is made from a material which easily flexes as needed during configuration of retractor 100 but returns to its original generally linear and/or straight orientation (see FIG. 1A) upon release of cables 106a and 106b. While tube 102 provides flexibility, when retractor 100 is in the second and/or assembled configuration, third, second and first sections 104a, 104b and 104c engage one another and provide retractor 100 with a degree of rigidity. In further embodiments, tube 102 may be omitted.

In a further embodiment of a retractor, a first section having a mechanical interface at a proximal end is disposed at a distal end of the retractor. A second section is disposed proximal to the first section. The second section has a second mechanical interface for engaging the first mechanical interface on the first section. A cable extends through the first and second sections and is offset from the longitudinal axis of the first and second sections. The second mechanical interface is disposed on a side surface of the second section and the cable is arranged so that upon pulling the cable in a proximal direction, the first mechanical interface is engaged with the second mechanical interface and the first section is disposed at an angle with a longitudinal axis of the retractor..

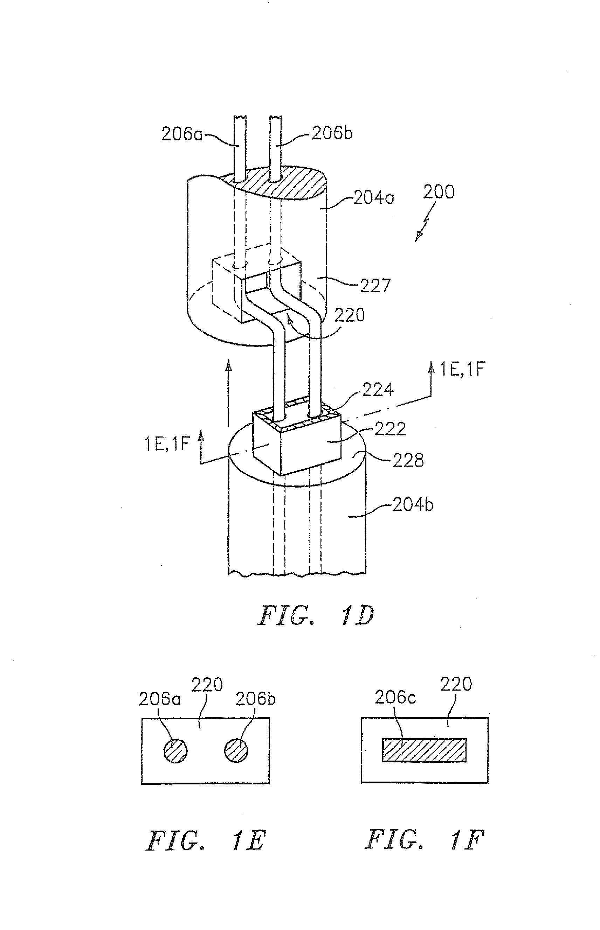

In a further embodiment shown in FIGS. 1D and 1E, a retractor 200 has a first section 204a and a second section 204b. As seen in FIG. 1D, first mechanical interface 220 is a socket for receiving second mechanical interface 222, which includes a tongue-like fitting disposed on and/or extending from a proximal surface 228 of second section 204b. Mechanical interface 220 is milled or otherwise formed, in a side surface 227 of first section 204a. It is envisioned that second mechanical interface 222 includes a chamfered edge 224 about the top periphery thereof which facilitates engagement with first mechanical interface 220 during assembly of sections 204a and 204b. One or more cables extend through both second mechanical interface 222 and first mechanical interface 220. Preferably, two cables 206a and 206b are provided for stability. Accordingly, when the surgeon actuates (e.g., pulls on) at least one of cables 206a, 206b, second section 204b is pulled towards first section 204a and second mechanical interface 222 inter-engages with first mechanical interface 220 to secure first and second sections 204a and 204b to one another for retraction purposes. Two or more such sections may be provided. It is envisioned that retractor 200 includes a flexible tube, like that shown in FIGS. 1A - 1C. However, the flexible tube may be omitted.

In an alternate embodiment, as seen in cross-section in FIG. 1F, cables 206a, 206b are replaced with at least one ribbon-like cable 206c extending through each of second mechanical interface 222 and first mechanical interface 220.

As can be appreciated, cables 206a, 206b and/or ribbon-like cable 206c effectuates translation of first section 204a and second section 204b relative to one another during the engagement and/or disengagement process for retractor 200. A third section (not shown) may be provided in further embodiments.

In a further embodiment shown in FIGS, 1G and 1H, a retractor 300 has a first section 304a and a second section 304b. In particular, first section 304a includes a first mechanical interface 320 including a helical earn-like surface 340a which engages a complementary cam-like surface 340b forming a second mechanical interface 322 for second section 304b. Accordingly, in use, surfaces 34E3a, 340b engage one another and rotate the first and second section 304a, 304b in the direction of arrows "R" upon translation of first section 304a and second section 304b towards one another in the direction of arrows "T".

A cable 306 is disposed along a central axis defined through each of first and second sections 304a and 304b and is utilized to effectuate translation and rotation of first section 304a and second section 304b relative to one another. As can be appreciated, surfaces 340a and 340b secure first section 304a and second section 304b in an interlocking, friction-fit manner for retraction purposes. Additionally or alternatively, cable 306 is secured to fix the relative positions of first section 304a and 304b. To disassemble retractor 300, as seen in FIG. 1H, the tension on the cable 306 is relaxed causing the sections to freely rotate and move away from one another in directions opposite to arrows "T" and "R".

Preferably, at least one of first and second sections 304a, 304b are arcuate in shape and/or are provided with a slight bend. In this manner, when second section 304b is mated with first section 304a, retractor 300 has a bent and/or arcuate configuration. Two or more such sections 304 may be provided. Desirably, retractor 300 includes a flexible tube like that shown in FIGS. 1A - 1C. However, the flexible tube may be omitted.

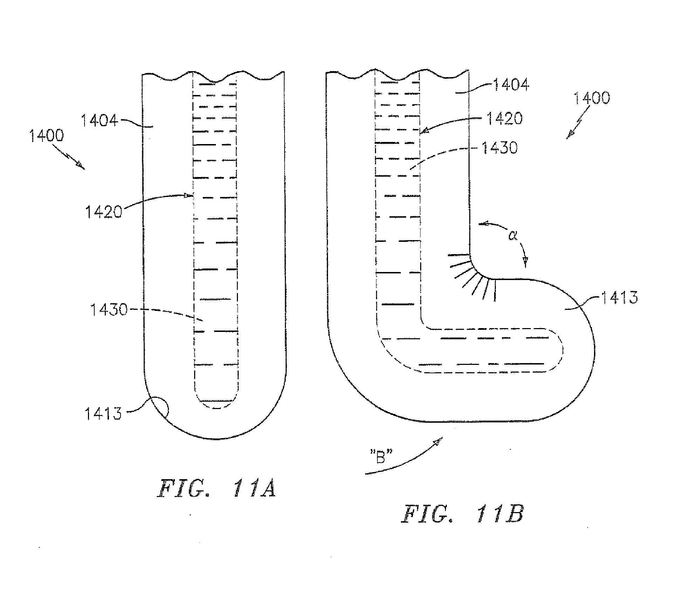

Turning now to FIG. 11, an enlarged perspective view of first section 404a for a retractor 400, in accordance with another embodiment of the present disclosure, is shown. First section 404a includes a surface 452, desirably angled with respect to longitudinal axis "A". Angled surface 452 defines an upper tip 453a and a lower tip 453b. Preferably, angled surface 452 includes first and second surfaces 452a, 452b, each angled with respect to an axis "A₁" extending through upper and lower tips 453a, 453b.

Second section 404b preferably includes a surface (not shown) which complements angled surfaces 452a, 452b of first section 404a. Accordingly, in use, when a cable (not shown) extending through a passage 455 of first section 404a is actuated remotely by a surgeon, to approximate second section 404b to first section 404a (as seen in FIG. 1J), surfaces 452a, 452b inter-engage the complementary surfaces of second section 404b to secure sections 404a and 404b at an angle with respect to one another for retraction purposes. Two or more such sections 404 may be provided. Desirably, retractor 400 includes a flexible tube like that shown in FIGS. 1A - 1C. However, the flexible tube may be omitted.

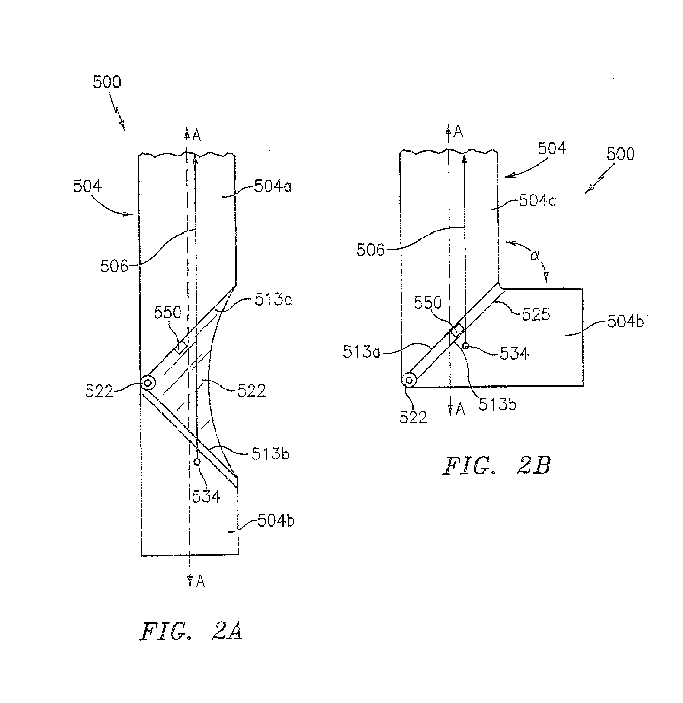

Turning now to FIGS. 2A and 2B, another embodiment of an endoscopic retractor in accordance with the present disclosure, is shown generally as 500. Organ retractor 500 includes an elongated shaft 504 having first and second sections 504a and 504b, respectively, pivotably connected to one another by a pivot member, preferably a hinge 522. Preferably, a film 525 extends between first and second sections 504a and 504b to reduce the susceptibility of organs and/or tissue from being pinched and/or caught between first and second sections 504a, 504b. Alternatively, first and second sections 504a, 504b may be covered by a flexible tube, as discussed above in connection with FIGS. 1A-1C. First and second sections 504a and 504b are rotatable relative to longitudinal axis "A" defined therethrough and about hinge 522 upon remote actuation of cable 506 by the surgeon. More particularly, as seen in FIG. 2B, cable 506 is affixed to second section 504b at point 534 such that selective translation (e.g., pulling) of cable 506 rotates second section 504b about pivot point 522. As can be appreciated, second section 504b may be rotated to various angles "a" relative to longitudinal axis "A" depending upon a particular purpose and depending on the particular dimensions and configuration of opposing surfaces 513a, 513b of first and second sections 504a and 504b, respectively. One or both of first and second sections 504a, 504b, respectively, may include a stop member 550 for limiting the degree of angular rotation "a" of second section 504b relative to first section 504a depending upon the particular purpose or to achieve a desired result.

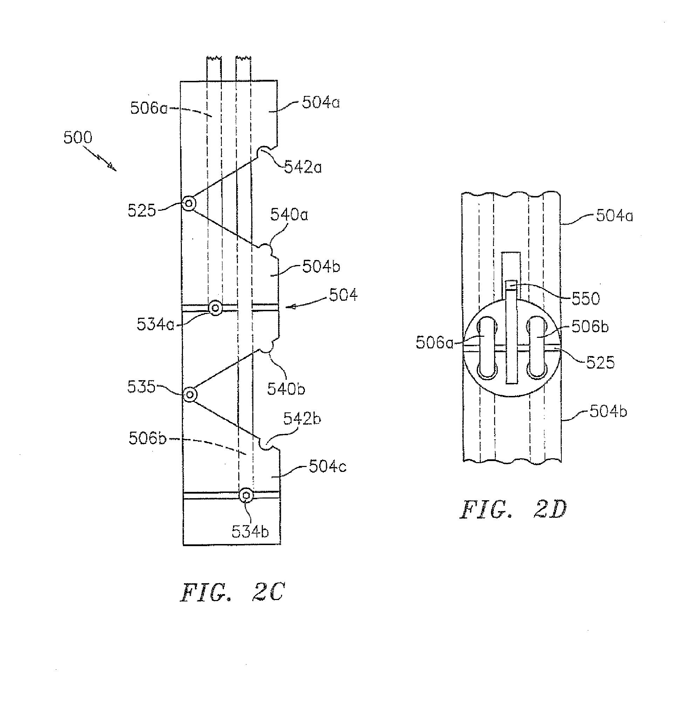

As seen in FIG. 2C, shaft 504 of organ retractor 500 can include three sections, namely, proximal section 504a, intermediate section 504b, and distal section 504c. Proximal and intermediate sections 504a and 504b are pivotably connected about hinge 525 while intermediate and distal sections 504b and 504c are pivotably connected about a hinge 535. A first cable 506a is affixed to intermediate section 504b at point 534a and a second cable 506b is affixed to distal section 504c at a point 534b. Much like the aforementioned embodiments, cables 506a and 506b allow the surgeon to remotely assemble retractor 500 for manipulation of organs. More particularly, actuation of cable 506b rotates distal section 504c about pivot 535 such that a proximal surface thereof contacts and/or otherwise engages a distal surface of intermediate section 504b. Actuation of cable 506a rotates intermediate section 504b about pivot 525 such that a proximal surface thereof contacts and/or otherwise engages a distal surface of proximal section 504a.

It is envisioned that one or more sections 504a, 504b, or 504c may include a series of mechanical interfaces 540a, 540b, 542a and 542b which facilitate engagement and alignment of sections 504a, 504b, or 504c during configuration and/or assembly of retractor 500. For example intermediate section 504b may include detents 540a and 540b which engage a complementary socket 542b disposed in distal section 504c and a complementary socket 542a disposed in proximal section 514a, respectively, upon formation of retractor 500. Alternatively, as seen in FIG. 2D, a tongue 550 may be utilized between proximal and intermediate sections 504a and 504b to assure proper and consistent rotation of intermediate section 504b relative to proximal section 504a during formation and/or configuration of retractor 500.

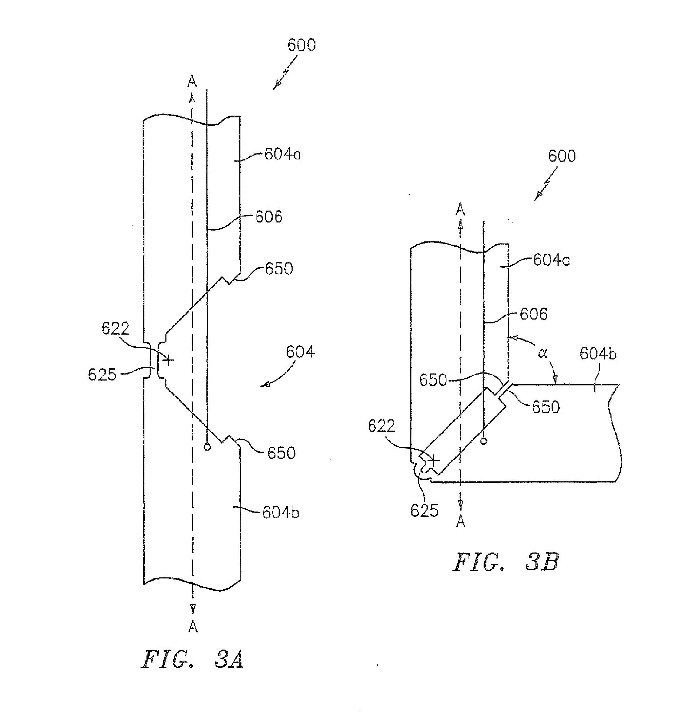

Turning now to FIGS. 3A and 3B, an endoscopic organ retractor, in accordance with yet another embodiment of the present disclosure, is designated generally as 600. Retractor 600 includes an elongated shaft 604 having first and second sections 604a and 604b, respectively, interconnected by a "living hinge" 625. A "living hinge" is a relatively thin portion of plastic or the like that bridges two relatively heavier and/or thicker walls and that provides the ability to repeatedly flex without the use of a mechanical hinge. First and second sections 604a, 604b are rotatable relative to a longitudinal axis "A" defined therethrough and about an imaginary pivot point 622 upon remote actuation of cable 606 by the surgeon. In particular, as seen in FIG. 3B, cable 606 is affixed to second section 614b at point 634 such that selective translation (e.g., pulling) of cable 606 biases second section 604b against living hinge 625 during rotation of second section 604b about imaginary pivot point 622. As can be appreciate, second section 604b may be rotated to various angles "a" relative to longitudinal axis "A" depending upon a particular purpose. One or both of first and second sections 604a, 604b, respectively, may include a stop member 650 for limiting the degree of angular rotation "a" of second section 604b depending upon a particular purpose or to achieve a desired result.

In the embodiments of FIGS. 2A-3B, the retractor may include a film extending between the sections, a flexible tube enclosing the sections of the retractor, or these feature may be omitted. In each of these embodiments, two or more sections may be provided in the retractor.

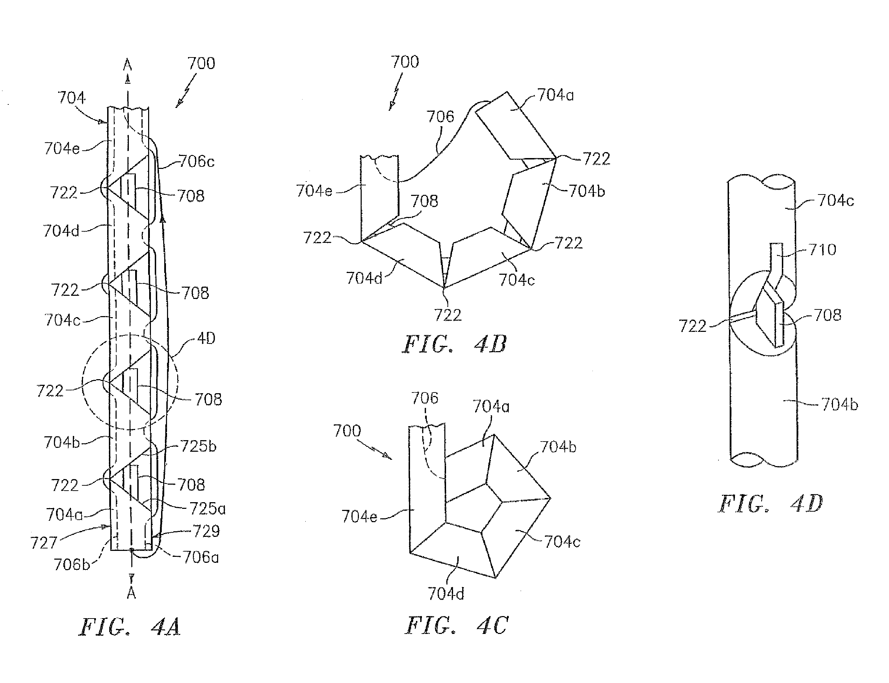

Turning now to FIGS. 4A - 4D, another embodiment of an endoscopic retractor in accordance with the present disclosure, is shown generally as 700. Retractor 700 includes an elongated shaft 704 having a plurality of sections 704a-704e pivotably connected to one another by a pivot member 722, preferably a hinge (e.g., a mechanical hinge, a living hinge, etc.). Retractor 700 has a first section 704a, a second section 704b, a third section 704c, a fourth section 704d and a fifth section 704e. However, people of ordinary skill in the field will appreciate that fewer or more sections may be used. Retractor 700 includes a cable 706 extending through the side of fifth section 704e, along the exterior of sections 704a-704d, and secured to the exterior surface of first section 704a. Each of sections 704 have a proximal end 725 and a distal end 728 that are angled, as opposed to obliquely oriented, with respect to longitudinal axis "A". As best seen in FIG. 4A, each section 704 has an angled distal end 728 that diverges from an angled proximal end 725 of an adjacent section. For example, distal end 728b (of section 704b) diverges from proximal end 725a (of section 704a), from a first side 727 to a second side 729 of retractor 700. The angled surfaces allow each section to rotate with respect to an adjacent section.

Sections 704a-704e are rotatable relative to longitudinal axis "A" defined therethrough and about pivot members 722 upon remote actuation of cable 706a by the surgeon. In particular, as cable 706a is drawn in a proximal direction, first section 704a is pulled towards and around to fifth section 704e, as seen in FIG. 4B. Preferably, cable 706c is drawn in a proximal direction until first section 704a contacts and/or rests against fifth section 704e. It is envisioned that the opposing surfaces of sections 704a-704e are angled an amount sufficient to enable first section 704a to contact fifth section 704e when retractor 700 is in a fully retracted condition.

As best seen in FIGS. 4A and 4D, each of sections 704a-704d of retractor 700 preferably include a tongue 708 extending therefrom in a direction for cooperative engagement with a complementary slot 710 formed in the adjacent section 704b-704e. For example, section 704b, has a tongue 708 at its proximal end, for engaging a slot 710 in the distal end of the third section 704c. Accordingly, when retractor 700 is in the fully retracted condition, as seen in FIG., 4C, tongues 708, in cooperation with slots 710, provide retractor 700 with increased rigidity and reduced susceptibility to twisting.

Retractor 700 further includes cable 706a extending through each section 704 and disposed between longitudinal axis "A" and second side 729 of the retractor 700. Cable 706b extends through each section 704 and is disposed between longitudinal axis "A" and first side 727. Preferably, cables 706a and 706b exit each section at a distal end of the section and enter the adjacent section at a proximal end of the section, as best seen in FIG. 4A. To bring retractor 700 from the first configuration (see FIG. 4A) to the second configuration (see FIG. 4C), cable 706a is pulled proximally, turning sections 704 about pivot members 722. To return retractor 700 to the first configuration, cable 706b is pulled proximally. In further embodiments, cable 706c may be provided without cables 706a and 706b. In other embodiments, cables 706a. and 706b are provided without cables 706c. Desirably, retractor 700 includes a flexible tube like that shown in FIGS. 1A - 1C, however, the flexible tube may be omitted.

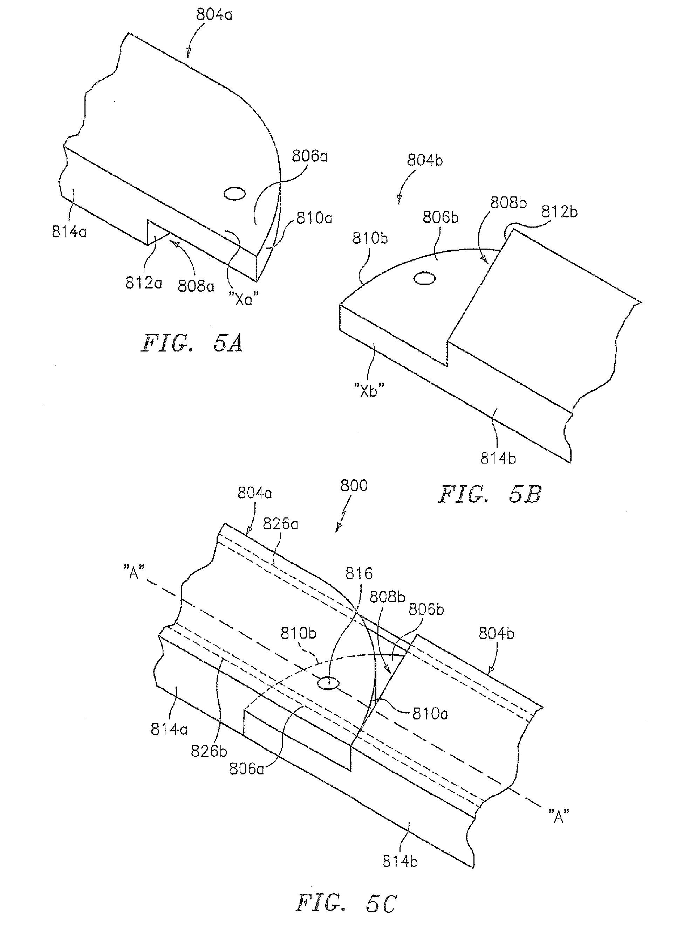

Turning now to FIGS. 5A - 5C, a segment of an endoscopic organ retractor 800, in accordance with another embodiment of the present disclosure, is shown. Retractor 800 includes at least a first section 804a and a second section 804b pivotably coupled to one another. As seen in FIG. 5A, first section 804a includes a tab, tongue or the like 806a extending longitudinally from a distal end 812a thereof. Tongue 806a defines a recess and/or cut-out 808a at the distal end of first section 804a. Tongue 806a includes an arcuate distal edge 810a. Arcuate distal edge 810a has a radius whose center "Xₐ" is desirably located at the intersection of a first side edge 814a of first section 804a and distal end 812a. As seen in FIG. 5B, second section 804b includes a tab, tongue or the like 806b extending longitudinally from a distal end 812b thereof. Tongue 806b defines a recess and/or cut-out 808b at the distal end of second section 804b. Tongue 806b includes an arcuate distal edge 810b. Arcuate distal edge 810b has a radius whose center "X_{b}" is desirably located at the intersection of a first side edge 814b of second section 804b (first side edge 814b being substantially aligned with first side edge 814a when first and second sections 804a, 804b are coupled together) and distal end 812b.

As seen in FIG. 5C, first and second sections 804a, 804b are pivotably coupled together by a pivot member 816 (e.g., a pivot pin) extending through tongues 806a, 806b. Preferably, when first and second sections 804a, 804b are coupled together, first side edge 814a is substantially aligned with first side edge 814b. In addition, arcuate distal edges 810a, 810b of tongues 806a, 806b, preferably over lap one another such that tongue 806a is disposed in recess 808b and tongue 806b is disposed in recess 808a. As best seen in FIG. 5C, desirably, distal edge 810a of tongue 806a contacts or lies adjacent to distal end 812b of second section 804b and distal edge 810b of tongue 806b contacts or lies adjacent to distal end 812a of first section 804a.

In this manner, retractor 800 is pivotable about pivot member 816 from a first position in which first section 804a is substantially longitudinally aligned with second section 804b, and any number of second positions, in which first section 804a is angled with respect to second section 804b. Tongues 806a, 806b inter-engage distal ends 812b, 812a in such a manner that first and second sections 804a, 804b pivot about pivot member 816 in a direction away from first side edges 814a, 814b. It is envisioned that retractor 800 can include a cable 826a extending through first section 804a and operatively connected to second section 804b. Cable 826a is offset from longitudinal axis "A" in such a manner so as to impart movement (i.e., pivoting) of second section 804b relative to first section 804a upon a pulling of cable 826a in a proximal direction, moving retractor 800 to the second configuration. Pulling of second cable 826b, which is disposed on an opposite side of axis "A" from cable 826a, returns retractor 800 to the first configuration. Alternatively, the second cable 826b may be omitted and the retractor may be retuned to the first configuration by releasing first cable 826a and allowing the sections to move under the force of gravity. Two or more of such sections 804 may be provided to form an L-shaped retractor, as shown in FIG. 1C or a loop, as shown in FIG. 4C. A flexible tube, like that shown in FIGS. 1A - 1C, is desirably included in retractor 800. However, the flexible tube, like that shown in FIGS. 1A - 1C, maybe omitted.

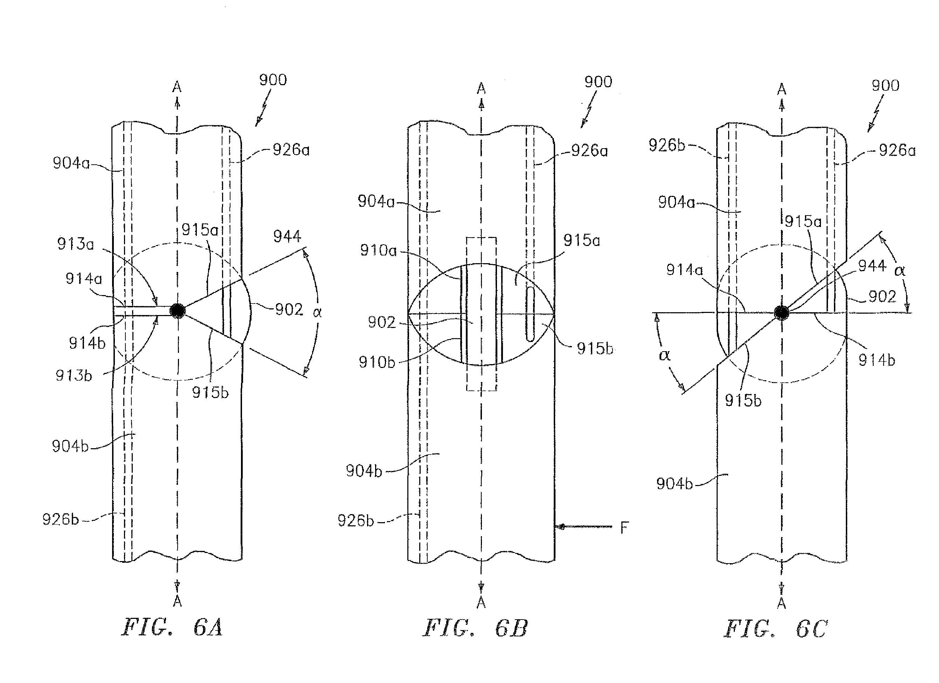

Turning now to FIGS. 6A - 6C, a segment of an endoscopic retractor 900, in accordance with another embodiment of the present disclosure, is shown. Retractor 900 includes at least a first section 904a and a second section 904b pivotably coupled to one another by a pivot member 944. As seen in FIGS. 6A and 6B, retractor 900 further includes a disc, wheel or the like 902 operatively disposed between first and second sections 904a, 904b so that the first section 904a is pivotably coupled with second section 904b.

First section 904a includes a distal surface 913a having a first surface 914a which is orthogonally oriented with respect to longitudinal axis "A" and a second surface 915a which is angled with respect to longitudinal axis "A". Second section 904b includes a proximal surface 913b having a first surface 914b which is orthogonally oriented with respect to longitudinal axis "A" and a second surface 915b which is angled with respect to longitudinal axis "A". Preferably, the central axis of disc 902 is orthogonally oriented with respect to longitudinal axis "A" and is positioned substantially at the intersection of first surfaces 914a, 914b and second surfaces 915a, 915b. Disc 902 is positioned within recesses 910a and 910b formed in first and second sections 904a, 904b, respectively. Disc 902 provides retractor 900 with a degree of rigidity when acted on by forces acting in a direction substantially parallel to the central axis of disc 902, as indicated by arrow F in FIG. 6B.

In one embodiment, as seen in FIGS. 6A and 6B, first surface 914a of first section 904a is juxtaposed relative to first surface 914b of second section 904b and second surface 915a of first section 904a is juxtaposed relative to second surface 915b of second section 904b. In this manner, retractor 900 is pivotable about the central axis of disc 902 from a first position in which first and second sections 904a, 904b are substantially aligned with one another and any number of second positions in which first and second sections 904a, 904b are pivoted about the central axis of disc 902 in order to be angled with respect to one another. First surfaces 914a, 914b engage one another and prevent first and second sections 904a, 904b from pivoting in a direction towards first and second surfaces 914a, 914b, Moreover, the angle of second surfaces 915a, 915b determines the angle and/or degree "a" to which retractor 900 can be bent.

Alternatively, as seen in FIG. 6C, first surface 914a of first section 904a is juxtaposed relative to second surface 915b of second section 904b and second surface 915a of first section 904a is juxtaposed relative to first surface 914b of second section 904b. In this manner, retractor 900 is pivotable about the central axis of disc 902 from a first position in which first and second sections 904a, 904b are substantially aligned with one another and any number of second positions in which first and second sections 904a, 904b are pivoted about the central axis of disc 902 in order to be angled with respect to one another. The position of second surfaces 915a, 915b of first and second sections 904a, 904b enables retractor 900 to be bent by an angle and/or degree "a" to either side thereof (i.e., in the direction of first surface 914a or in the direction of second surface 915a).

It is envisioned that retractor 900 includes a cable 926a extending through first section 904a and operatively connected to second section 904b. Cable 926a is offset from longitudinal axis a in such a manner so as to impart movement (i.e., pivoting) of second section 904b relative to first section 904a upon a pulling of cable 926 in a proximal direction, moving retractor 900 to the second configuration. A second cable 926b is offset from longitudinal axis "A" in a second direction from cable 926a, so that pulling cable 926b returns retractor 900 to the first configuration. Alternatively, the second cable 926b may be omitted and the retractor may be returned to the first configuration by releasing the first cable 926a and allowing the sections to move under the force of gravity. Retractor 900 may include two or more of the sections 904, to provide an L-shaped retractor as shown in FIG. 1C, or a loop-shaped retractor as shown in FIG. 4C. Retractor 900 may also include a flexible tube, like that shown in FIGS. 1A - 1C. However, the flexible tube may be omitted.

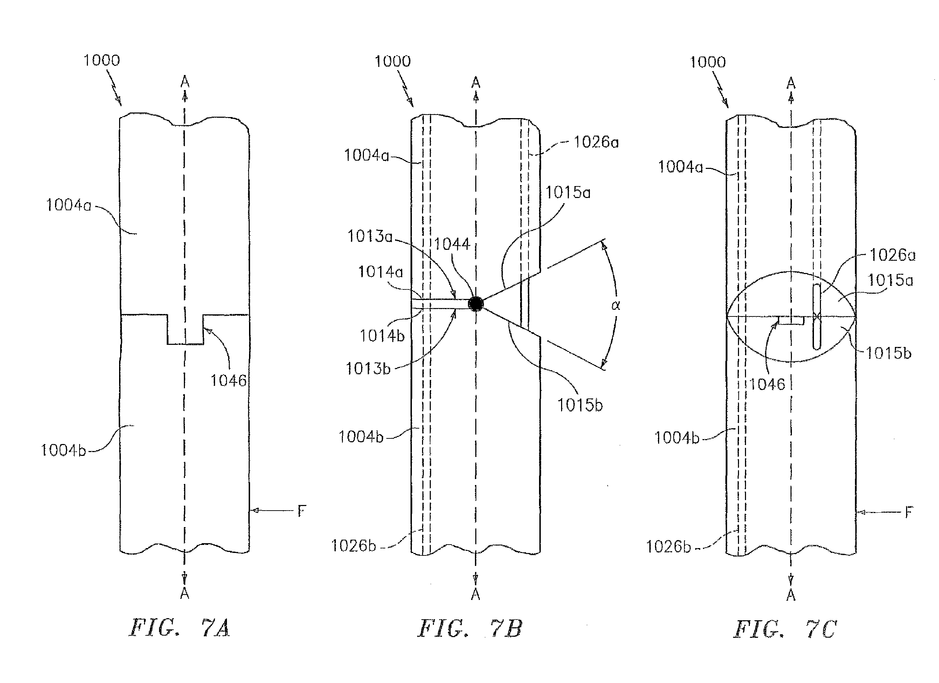

Turning now to FIGS. 7A - 7C, a segment of an endoscopic retractor 1000, in accordance with another embodiment of the present disclosure, is shown. Retractor 1000 includes at least a first section 1004a and a second section 1004b pivotably coupled to one another by a pivot member 1044. As seen in FIG. 7A, first and second sections 1004a, 1004b of retractor 1000 are joined together by a knuckle joint 1046 (e.g., tongue and groove, dove-tail, etc.).

First section 1004a includes a distal surface 1013a having a first surface 1014a which is orthogonally oriented with respect to longitudinal axis "A" and a second surfaces 1015a which is angled with respect to longitudinal axis "A", Second section 1004b includes a proximal surface 1013b having a first surface 1014b which is orthogonally oriented with respect to longitudinal axis "A" and a second surface 1015b which is angled with respect to longitudinal axis "A". The angle of second surfaces 1015a, 1015b determines the angle and/or degree "a" to which retractor 1000 can be bent. Joint 1046 provides retractor 1000 with a degree of rigidity when acted on by forces acting in a direction substantially parallel to the axis of rotation of first and second sections 1004a, 1004b, as indicated by arrow F in FIGS. 7A and 7C.

It is envisioned that retractor 1000 includes a cable 1026a extending through first section 1004a and operatively connected to second section 1004b. Cable 1026a is offset from longitudinal axis "A" in such a manner so as to impart movement (i.e., pivoting) of second section 1004b relative to first section 1004a upon a pulling of cable 1026a in a proximal direction, moving retractor 1000 to the second configuration. A second cable 1026b is offset from axis "A" in an opposite direction from cable 1026a, so that pulling on cable 1026b returns retractor 1000 to the first configuration. Alternatively, the second cable 1026b may be omitted and the retractor is returned to the first configuration by releasing the first cable 1026a and allowing the sections to move under the force of gravity. Retractor 1000 may include two or more sections 1004, to provide an L-shaped retractor as shown in FIG. 1C, or a loop-shaped retractor, as shown in FIG. 4C. Desirably, retractor 1000 includes a flexible tube like that shown in FIGS. 1A - 1C. However, the flexible tube may be omitted.

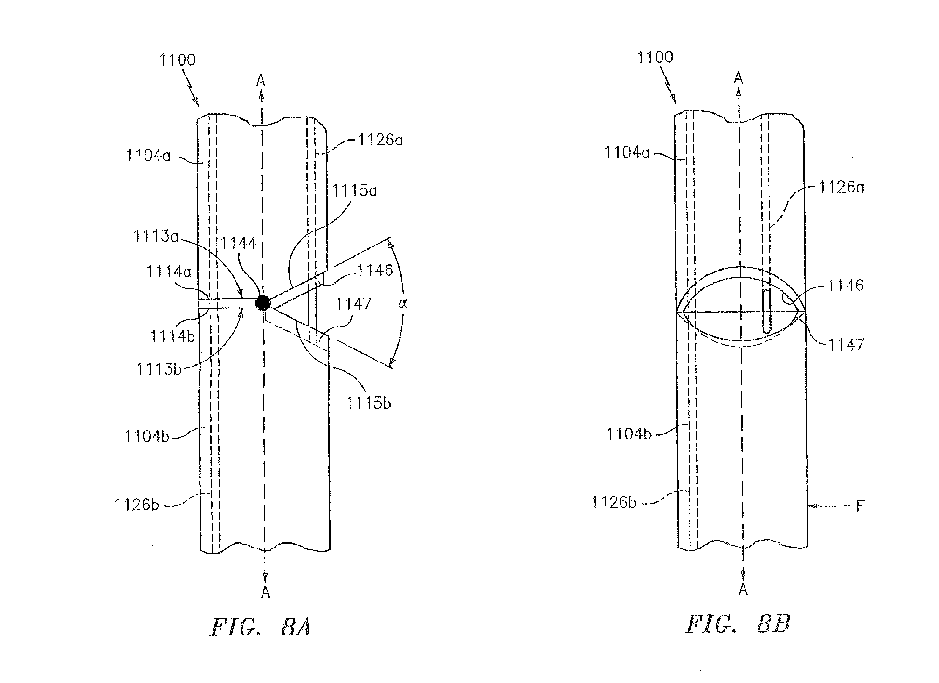

Turning now to FIGS. 8A and 8B, a segment of an endoscopic retractor 1100, in accordance with another embodiment of the present disclosure, is shown. Retractor 1100 includes at least a first section 1104a and a second section 1104b pivotably coupled to one another by a pivot member 1144. First section 1104a includes a distal surface 1113a having a first surface 1114a which is orthogonally oriented with respect to longitudinal axis "A" and a second surface 1115a which is angled with respect to longitudinal axis "A". Second section 1104b includes a proximal surface 1113b having a first surface 1114b which is orthogonally oriented with respect to longitudinal axis "A" and a second surface 1115b which is angled with respect to longitudinal axis "A", The angle of second surfaces 1115a, 1115b determines the angle and/or degree "a" to which retractor 1100 can be bent.

Retractor 1100 further includes a tab 1146 extending from one of second surface 1115a of first section 1104a or second surface 1115b of second section 1104b. Retractor 1100 further includes a recess or depression 1147 formed in the other of second surface 1115a of first section 1104a and second surface 1115b of second section 1104b. Preferably, tab 1146 is complementary in shape to recess 1147. Tab 1146 and recess 1147 provide retractor 1100 with a degree of rigidity, when in the bent configuration, when acted on by forces acting in a direction substantially parallel to the axis of rotation of first and second sections 1104a, 1104b, as seen in FIG. 8B.

It is envisioned that retractor 1100 includes a cable 1126a extending through first section 804a and operatively connected to second section 1104b. Cable 1126a is offset from longitudinal axis "A" in such a manner so as to impart movement (i.e., pivoting) of second section 1104b relative to first section 1104a upon a pulling of cable 1126 in a proximal direction to move retractor 1100 to the second configuration. Retractor 1100 has a second cable 1126b offset from axis "A" in an opposite direction from cable 1126a so that pulling on cable 1126b returns retractor 1100 to the first configuration. Alternatively, the second cable 1126b may be omitted and the retractor is returned to the first configuration by releasing the first cable 1126a and allowing the sections to move under the force of gravity. Retractor 1100 may include two or more sections 1104 to provide an L-shaped retractor, as shown in FIG. 1C, or a loop-shaped retractor, as shown in FIG. 4C. Desirably, retractor 1100 includes a flexible tube, as shown in FIGS. 1A - 1C. However, the flexible tube may be omitted.

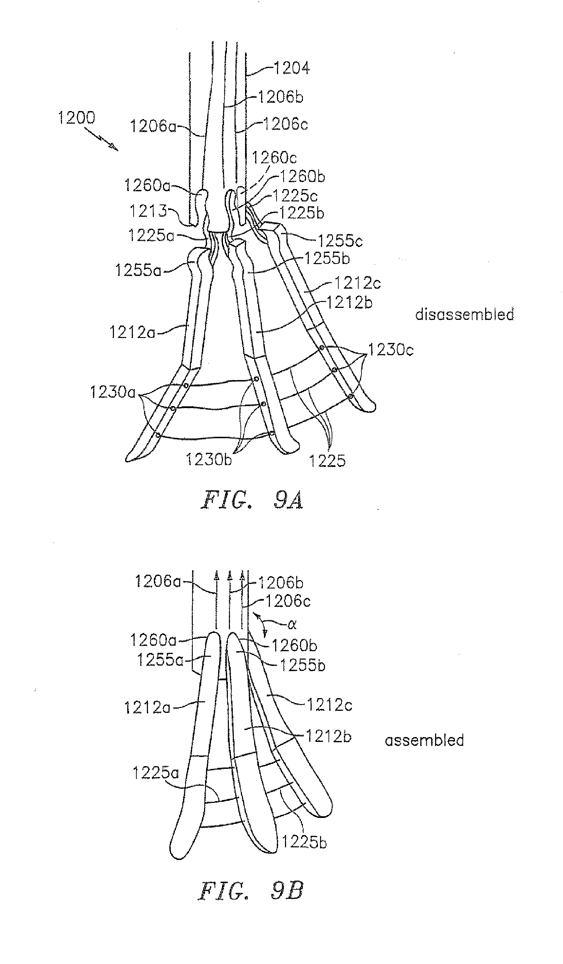

Turning now to FIGS. 9A and 9B, an endoscopic retractor, in accordance with yet another embodiment of the present disclosure, is generally designated as retractor 1200, Retractor 1200 includes an elongated shaft 1204, and a plurality of finger elements 1212a, 1212b and 1212c which extend from and are operatively engagable with a distal end 1213 of shaft 1204. Retractor 1200 also includes a plurality of cables 1206a, 1206b and 1206c disposed therethrough which are remotely operable by the surgeon to form, assemble and/or configure retractor 1200 after insertion through a trocar assembly (not shown). Each cable 1206a-1206c includes a bundle of cords 1225a-1225c which extend from a respective cable 1206a-1206c and into a corresponding finger element 1212a-1212c. Each bundle of cords 1225a-1225c, in turn, separates into individual cord elements (not shown) which ultimately connect to and/or inter-connect adjacent finger elements 1212a-1212c to one another through a series of side ports 1230a-1230c formed in each finger element 1212a-1212c, respectively.

As best illustrated in FIG. 9B, after the surgeon inserts retractor 1200 through the trocar assembly (not shown), the surgeon simply pulls cables 1206a, 1206b and 1206c in a proximal direction to form the supporting structure of retractor 1200. In particular, by pulling cables 1206a-1206c in a proximal direction, the corresponding bundle of cords 12925a-1225c are also pulled proximally which, in turn, pull finger elements 1212a-1212c into engagement with distal end 1213 of shaft 1244 and pull the adjacent finger elements 1212a-1212c, into tight cooperation with one another to facilitate organ retraction. As can be appreciated, cables 1206a-1206c can be actuated simultaneously or sequentially depending upon a particular purpose.

As best seen in FIG. 9A, it is envisioned that distal end 1213 of shaft 1214 may include a series of key-like sockets 1260a, 1260b and 1260c which mate with a corresponding flanges 1255a, 1255b and 1255c formed at a proximal end of each finger element 1212a-1212c, respectively. Each flange 1255a-1255c may be shaped to interface with a corresponding socket 1260a-1260c such that the corresponding finger element 1212a-1212c, when engaged with distal end 1213 of shaft 1214, is disposed at a particular angle "a" relative to a longitudinal axis of shaft 1214 in order to facilitate retraction and handling of a body organ.

Each finger element 1212a-1212c may comprise a plurality of sections having inter-engaging interfaces and a plurality of cables (e.g., 1206a-1206c) for articulating the sections with respect to one another. The inter-engaging interfaces may comprise any pair of complementary shapes on adjacent sections. The sections may be connected by a living hinge or mechanical hinge, or may be unconnected, as discussed above. Desirably, finger elements 1212a-1212c articulate with respect to shaft 1204 by operation of cables 1206a-1206c,

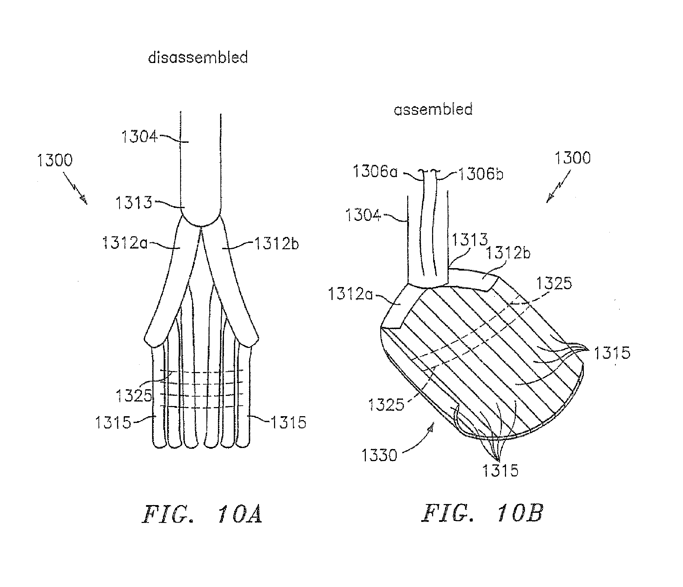

Turning now to FIGS. 10A and 10B, an endoscopic retractor, in accordance with yet another embodiment of the present disclosure, is generally designated 1300. Retractor 1300 includes a shaft 1304, and at least a pair of cables 1306a and 1306b disposed therethrough. Cables 1306a, 1306b are remotely operable by a surgeon to assemble and disassemble retractor 1300 as needed during surgery. Retractor 1000 further includes a pair of arms and/or plates 1312a and 1312b pivotably affixed to a distal end 1313 of shaft 1314. Plates 1312a, 1312b are movable from a first orientation (i.e., having a reduced-diameter, as seen in Fig. 10A, in which finger elements 1315 are substantially aligned with the longitudinal axis, to facilitate insertion through a trocar assembly (not shown) to a second orientation (i.e., expanded), in which finger elements 1315 are at an angle with respect to the longitudinal axis, to facilitate retraction of tissue and organs during surgery (see Fig. 10B). As seen in FIG. 10B, each plate 13122a and 312b includes a plurality of finger elements 1315 affixed thereto which, when plates 1312a and 1312b are expanded to the second configuration, mutually cooperate to form a scoop-like structure or trowel 1330, which enhances organ retraction. It is envisioned that the plurality of finger elements 1315 are connected to one another by a series of wires 1325 (or the like) which finger elements 1315 become rigid upon expansion of the plates 1312a and 1312b to provide additional support for scoop-like structure 1330.

Turning now to FIGS. 11A and 11B, an endoscopic retractor, in accordance with an alternate embodiment, is generally designated as 1400. Retractor 1400 includes an elongated shaft 1404 having a bore, lumen and/or elongate recess 1420 formed therethrough and which contains a liquid 1430 retained in recess 1420 thereof. A distal end 1413 of shaft 1414 is preferably made from a shape memory alloy such that upon a change in temperature of liquid 1430 within recess 1420, distal end 1413 of shaft 1414 transform and/or configures into a scoop-like configuration, as indicated by arrow "B", for retracting organs. It is envisioned that distal end 1413 can be configured to have any angle "a" corresponding to a specific purpose or to achieve a particular result.

Shape memory alloys (SMAs) transform in shape when changed from an austenitic state to a martenistic state due to a change in temperature. SMAs are a family of alloys having anthropomorphic qualities of memory and trainability and are particularly well suited for use with medical instruments. SMAs have been applied to such items as actuators for control systems, steerable catheters, and clamps. One of the most common SMAs is Nitinol which can retain shape memories for two different physical configurations and changes shape as a function of temperature.

Recently, other SMAs have been developed based on copper, zinc and aluminum and have similar shape memory retaining features. SMAs undergo a crystalline phase transition upon applied temperature and/or stress variations. A particularly useful attribute of SMAs is that after it is deformed by temperature/stress, it can be completely recover to its original shape upon its return to the original temperature. This transformation is referred to as a thermoelastic martenistic transformation.

Under normal conditions, the thermoelastic martenistic transformation occurs over a temperature range which varies with the composition of the alloy, itself, and the type of thermal-mechanical processing by which it was manufactured. In other words, the temperature at which a shape is "memorized" by an SMA is a function of the temperature at which the martensite and austenite crystals form in that particular alloy. For example, Nitinol alloys can be fabricated so that the shape memory effect will occur over a wide range of temperatures, e.g., about -270 to about +100 degrees Celsius.

It is further envisioned that the shape memory alloy can be replaced with a shape memory plastic when forming an endoscopic organ retractor for use in manipulating organs. Shape memory plastics are polymeric materials which exhibit the property of shape memory similar to that of shape memory alloys.

It will be understood that various modifications may be made to the various embodiments shown herein. For example, the embodiments of the invention discussed above are directed to a retractor. In further embodiments of the invention, an instrument comprises an articulating shaft, including a plurality of sections having inter-engaging interfaces and a plurality of cables connected to the sections for articulating the sections with respect to one another. The instrument may comprise any instrument including a stapler, dissector, shears and the like for endoscopic and/or any other surgical technique. Therefore, the above description should not be construed as limiting, but merely as exemplifications of preferred embodiments. Those skilled in the art will envision other modifications within the scope and spirit of the claims appended hereto.

The following numbered items correspond to the claims of the parent application as originally published.
1. A retractor for use through a trocar port, comprising:
   a shaft having at least a first section having a first mechanical interface and a second section having a second mechanical interface for engaging the first mechanical interface, the first section and second section being selectively movable from a first, generally longitudinally-aligned configuration along an axis defined through the shaft and the first mechanical interface is disengaged from the second mechanical interface, to a second configuration wherein the second section is disposed at an angle relative to a longitudinal axis of the shaft and the first mechanical interface is engaged with the second mechanical interface; and
   at least one cable extending through the shaft and being operatively secured to the second section, the cable being remotely actuatable to move the second section from the first to the second configuration upon selective translation of the cable.
2. A retractor according to 1 wherein the first and second mechanical interfaces cooperate to align the first section and the second section and engage the first and second sections with one another upon movement from the first configuration to the second configuration.
3. A retractor according to 1 wherein the first section includes a cam-like interface and the second section includes a complementary cam-like interface which rotatably and translatably engage one another upon actuation of the cable and movement of the first section and the second section from the first configuration to the second configuration.
4. A retractor according to 1 wherein the shaft includes an outer sleeve which houses the first and second sections.
5. A retractor according to 1 wherein at least one of the first section and the second section include a tongue which engages a corresponding recess disposed within the other of the first section and the second section to facilitate alignment and engagement of the first section and the second section relative to one another during movement from the first configuration to the at least one additional second configuration.
6. A retractor according to 1, further comprising a hinge disposed between the first section and the second section.
7. A retractor according to 1 further comprising a living hinge disposed between the first section and the second section.
8. A retractor according to 6 wherein one of the first section and the second section includes a stop for controlling the angular disposition of the first section and the second section when disposed in the at least one additional second configuration.
9. An organ retractor, comprising:
   a tube having a lumen extending therethrough and defining a longitudinal axis;
      and
   a distal section, an intermediate section and a proximal section disposed within the lumen of the tube, wherein the retractor has a first configuration in which the distal, intermediate and proximal sections are substantially aligned with the longitudinal axis and disassociated with one another, and at least one second configuration in which the intermediate section and the distal section are engaged with one another so that the distal section is disposed at an angle with respect to the longitudinal axis.
10. The organ retractor of 9, further comprising a first cable extending through the proximal section and the intermediate section, and operatively secured to the distal section, wherein translation of the first cable in a proximal direction causes the distal section to operatively engage the intermediate section at an angle relative to the longitudinal axis.
11. The organ retractor of 10, further comprising a second cable extending through the proximal section and operatively secured to the intermediate section, wherein translation of at least one of the first and second cables in a proximal direction causes the intermediate section to operatively engage the proximal section.
12. The organ retractor of 11, wherein the distal section includes at least one first mechanical interface formed at a proximal end thereof and the intermediate section includes at least one second mechanical interface formed on a side surface thereof, the second mechanical interface being complementary with the first mechanical interface, wherein when the distal and intermediate sections engage one another, the first mechanical interface and the second mechanical interface maintain the distal section at an angle with respect to the longitudinal axis.
13. The organ retractor of 12, wherein the proximal section includes at least one third mechanical interface formed at a distal end thereof and the intermediate section includes at least one fourth mechanical interface formed at a proximal end thereof, the fourth mechanical interface being complementary to the third mechanical interface, wherein when the proximal and intermediate sections engage one another, the third mechanical interface and the fourth mechanical interface maintain the proximal and intermediate sections substantially aligned with the longitudinal axis.
14. The organ retractor of 13, wherein the proximal section includes at least one longitudinally oriented passage extending therethrough, wherein the first and the second cables extend through the at least one longitudinal passage.
15. The organ retractor of 14, wherein the intermediate section includes a substantially angular passage extending therethrough, a first portion of the angular passage opening on the proximal surface of the intermediate section, and a second portion of the angular passage opening on the side surface of the intermediate section, wherein the second cable extends through the angular passage.
16. The organ retractor of 12, wherein the second mechanical interface of the intermediate section is in the form of a socket and wherein the first mechanical interface of the distal section is in the form of a tongue-like member which extends therefrom and is complementary to the socket formed in the proximal section.
17. The organ retractor of 9, wherein a cable in the form of a ribbon extends through the proximal section and the intermediate section and is affixed to the distal section.
18. The organ retractor of 12, wherein the second mechanical interface of the intermediate section includes a helical camming surface and wherein the first mechanical interface of the distal section includes a helical camming surface which is complementary to the helical camming surface of the proximal section.
19. The organ retractor of 12, wherein the third mechanical interface of the proximal section and the fourth mechanical interface of the intermediate section each comprise a helical camming surfaces which intersect one another.
20. The organ retractor of 9, wherein the tube is fabricated from a flexible material.
21. An organ retractor, comprising:
   an elongated shaft defining a longitudinal axis, the shaft having a first section and a second section pivotably connected to one another; and
   a first cable extending through the first section and operatively connected to the second section for manipulating the retractor from a first configuration to at least one second configuration, wherein in the first configuration the first and second sections are substantially aligned with the longitudinal axis and in the at least one second configuration the second section is at an angle with respect to the longitudinal axis.
22. The organ retractor of 21, wherein the second section is pivotably connected to the first section by a mechanical hinge.
23. The organ retractor of 21, wherein the second section is pivotably connected to the first section by a living hinge.
24. The organ retractor of 21, wherein the first section has a distal surface and the second section has a proximal surface, the distal surface comprising an angled surface that faces the proximal surface of the second section.
25. The organ retractor of 24, further including a film extending between the first and second sections.
26. The organ retractor of 24, further including at least one stop member provided on at least one of the distal surface and the proximal surface.
27. The organ retractor of 21, further comprising:
   a third section pivotably connected to the second section; and
   a second cable extending through the first section and the second section and operatively connected to the third section for manipulating the retractor from the first configuration to the at least one second configuration.
28. The organ retractor of 27, further comprising a first mechanical interface provided on the first section, a second mechanical interface provided on the second section for engaging the first mechanical interface, a third mechanical interface provided on the second section, and a fourth mechanical interface on the third section for engaging the third mechanical interface.
29. An organ retractor, comprising:
   a shaft defining a longitudinal axis; and
   a plurality of finger elements operatively engagable with a distal end of the shaft, wherein the retractor has a first configuration in which the plurality of finger elements are substantially aligned with the longitudinal axis and at least one second configuration in which the plurality of finger elements are disposed at an angle with respect to the longitudinal axis.
30. The organ retractor of 29, wherein each of the plurality of finger elements is disassociated from the shaft, and wherein the retractor includes a plurality of cables extending through the shaft, each cable having a bundle of cords extending therefrom and into a corresponding finger element, each bundle of cords being operatively connected to the corresponding finger element such that retraction of the plurality of cables manipulates the retractor from the first configuration to the at least one second configuration.
31. The organ retractor of 30, wherein the bundle of cords extend between the plurality of finger elements.
32. The organ retractor of 31, wherein a distal end of the shaft includes a plurality of sockets configured and dimensioned to selectively receive a flange formed at a proximal end of a corresponding finger element.
33. The organ retractor of 32, wherein individual cords of the bundle of cords exit a respective finger element through ports formed therein.
34. The organ retractor of 29, further including a pair of plates pivotably connected to a distal end of the shaft and wherein the plurality of finger elements are affixed to the pair of plates, wherein the pair of plates have a first orientation in which the retractor is in the first configuration and a second orientation in which the retractor is in the at least one second configuration.
35. The organ retractor of 34, farther including at least one wire extending between adjacent finger elements.
36. An organ retractor, comprising:
   a shaft defining a longitudinal axis and a bore for receiving a temperature changing medium, the shaft being fabricated from a shape memory substance, wherein the shaft has a first configuration, which is substantially linear when at a first temperature and at least one second configuration which is non-linew when at a second temperature.
37. The organ retractor of 36, wherein the temperature changing medium comprises a quantity of liquid received in the bore.
38. The organ retractor of 36, wherein the shaft is fabricated from one of a shape memory alloy and a shape memory plastic.
39. The organ retractor of 36, wherein the shaft is fabricated from nitinol.
40. The organ retractor of 36, wherein the shaft will undergo a change of configuration from about -270°C to about +100°C.
41. The organ retractor of 3 7, wherein the liquid transmits a change of temperature to the shaft.
42. A retractor, comprising:
   a plurality of sections defining a shaft, each of the sections having a mechanical interface for engaging an adjacent section, each section having a first position in longitudinal alignment with an adjacent section and a second position offset from the first position so that the sections form a substantially closed shape for engaging tissue.
43. The retractor of 42, wherein at least one of the sections includes a tongue for engaging a slot in all adjacent section.
44. The retractor of 42, further comprising a first cable attached to at least a first section of the plurality of sections and disposed in a passage in at least a second section of the plurality of sections, and arranged for moving the first section with respect to a second section when the first cable is pulled in a proximal direction, the first cable being offset from a longitudinal axis of the shaft in a first direction.
45. The retractor of 44, further comprising a second cable offset from the longitudinal axis in a second direction, for returning the retractor to the first position.
46. The retractor of 42, further comprising a hinge disposed between a first section of the plurality of sections and a second section of the plurality of sections.
47. The retractor of 45, wherein the hinge comprises a living hinge.

## Claims

1. An organ retractor, comprising:
a shaft defining a longitudinal axis and a bore for receiving a temperature changing medium, the shaft being fabricated from a shape memory substance, wherein the shaft has a first configuration which is substantially linear when at a first temperature and at least one second configuration which is non-linear when at a second temperature.

2. The retractor of claim 1, wherein the temperature changing medium comprises a quantity of liquid received in the bore.

3. The retractor of claim 1, wherein the shaft is fabricated from one of a shape memory alloy and a shape memory plastic.

4. The retractor of claim 1, wherein the shaft is fabricated from nitinol.

5. The retractor of claim 1, wherein the shaft will undergo a change of configuration from -270°C to about +100°C.

6. The retractor of claim 2, wherein the liquid transmits a change of temperature to the shaft.

7. The retractor of claim 1, having a plurality of sections defining a shaft and including at least the first section, second section and third section, each section having a first position in longitudinal alignment with an adjacent section and a second position offset from the first position so that the sections form a substantially closed shape for engaging tissue.

8. The retractor of claim 7, wherein at least one of the sections includes a tongue for engaging a slot in an adjacent section.

9. The retractor of claim 7, further comprising a hinge disposed between at least the second section of the plurality of sections and the third section of the plurality of sections.

10. The organ retractor of claim 2, wherein the bore contains the liquid.

11. An organ retractor, comprising:
a shaft defining a longitudinal axis; and
a plurality of finger elements operatively engagable with a distal end of the shaft, wherein the retractor has a first configuration in which the plurality of finger elements are substantially aligned with the longitudinal axis and at least one second configuration in which the plurality of finger elements are disposed at an angle with respect to the longitudinal axis.

12. The organ retractor of claim 11, wherein each of the plurality of finger elements is disassociated from the shaft, and wherein the retractor includes a plurality of cables extending through the shaft, each cable having a bundle of cords extending therefrom and into a corresponding finger element, each bundle of cords being operatively connected to the corresponding finger element such that retraction of the plurality of cables manipulates the retractor from the first configuration to the at least one second configuration.

13. The organ retractor of claim 12, wherein the bundle of cords extend between the plurality of finger elements, preferably
wherein a distal end of the shaft includes a plurality of sockets configured and dimensioned to selectively receive a flange formed at a proximal end of a corresponding finger element, and preferably
wherein individual cords of the bundle of cords exit a respective finger element through ports formed therein.

14. The organ retractor of claim 11, further including a pair of plates pivotably connected to a distal end of the shaft and wherein the plurality of finger elements are affixed to the pair of plates, wherein the pair of plates have a first orientation in which the retractor is in the first configuration and a second orientation in which the retractor is in the at least one second configuration, preferably further including at least one wire extending between adjacent finger elements.

15. A method of operating an organ retractor, the organ retractor comprising:
a shaft defining a longitudinal axis; and
a plurality of finger elements operatively engagable with a distal end of the shaft, wherein the retractor includes a plurality of cables extending through the shaft, each cable having a bundle of cords extending therefrom and into a corresponding finger element, the retractor being in a first configuration in which the plurality of finger elements are substantially aligned with the longitudinal axis, wherein each of the plurality of finger elements is disassociated from the shaft, the method comprising retracting the plurality of cables to manipulate the retractor from the first configuration to at least one second configuration in which the plurality of finger elements are disposed at an angle with respect to the longitudinal axis.
